# EUROPÄISCHE PATENTSCHRIFT

(11) **EP 1 038 923 B1**
(45) Veröffentlichungstag und Bekanntmachung des Hinweises auf die Patenterteilung: **22.06.2005**
(21) Anmeldenummer: 00105281.0
(22) Anmeldetag: 14.03.2000
(51) Int. Cl.: C09B 19/02, C09B 67/10, C07D 498/22

(54) **Verfahren zur Herstellung von Dioxazin-Verbindungen**
Process for producing dioxazine compounds
Procédé de préparation de composés dioxaziniques

(30) Priorität: 25.03.1999 DE 19913401
(43) Veröffentlichungstag der Anmeldung: 27.09.2000
(73) Patentinhaber: Clariant GmbH, 65929 Frankfurt am Main (DE)
(72) Erfinder: Nagl, Gert, Dr., 61138 Niederdorfelden (DE); Bauer, Wolfgang, Dr., 63477 Maintal (DE); Kempter, Peter, Dr., 65812 Bad Soden (DE)

(56) Entgegenhaltungen:
- EP-A- 0 837 107
- US-A- 5 185 435
- PATENT ABSTRACTS OF JAPAN vol. 1996, no. 04, 30. April 1996 (1996-04-30) -& JP 07 331097 A (NIPPON KAYAKU CO LTD), 19. Dezember 1995 (1995-12-19)
- DATABASE WPI Section Ch, Week 198149 Derwent Publications Ltd., London, GB; Class E13, AN 1981-89901D XP002215641 & JP 56 135556 A (SUMITOMO CHEM CO LTD), 23. Oktober 1981 (1981-10-23)

## Beschreibung

Die vorliegende Erfindung liegt auf dem Gebiet der organischen Farbmittel und betrifft ein verbessertes Verfahren zur Herstellung von Dioxazin-Verbindungen. Dioxazin-Verbindungen werden zur Herstellung wertvoller Farbstoffe und Pigmente verwendet (s. Ullmann's Encyclopedia of Industrial Chemistry, Vol. A 3, p. 233). Sie werden technisch in einer fünfstufigen Synthese, die eine N-Alkylierung von Carbazol, Nitrierung, Reduktion, Kondensation und Ringschluß umfaßt, hergestellt. In der letzten Synthesestufe werden Verbindungen der allgemeinen Formel (II) 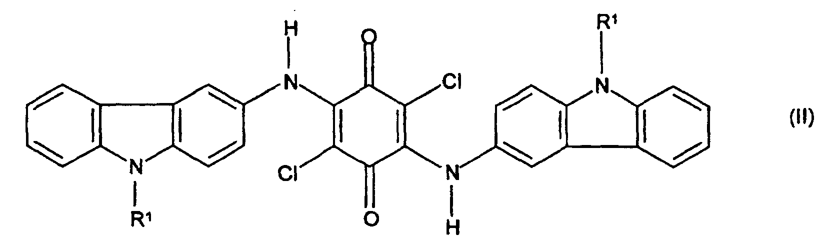 mit Ringschlußmitteln, wie z. B. Benzolsulfonsäurechlorid oder 4-Toluolsulfonsäurechlorid, zu den Dioxazin-Verbindungen cyclisiert (DE-A 30 10 949). Für den Ringschluß werden hohe Temperaturen benötigt. Deshalb kommen als Reaktionsmedium für den Ringschluß nur inerte Lösungsmittel infrage, deren Siedepunkt bei Normaldruck über 160°C liegt. Als solche wurden halogenhaltige aromatische Lösungsmittel, insbesondere o-Dichlorbenzol, ferner Nitrobenzol, polare aprotische Lösungsmittel, wie Chinolin, sowie halogenhaltige Monoalkyl-, Dialkyl-, Trialkylbenzole, Alkylnaphthaline und auch Alkane und Alkene vorgeschlagen. Von diesen Reaktionsmedien hat sich für die technische Herstellung von Dioxazin-Verbindungen bisher lediglich das o-Dichlorbenzol bewährt, da dieses Lösungsmittel in technischer und wirtschaftlicher Hinsicht vielerlei Vorteile bietet. Wesentliche Vorteile des o-Dichlorbenzols sind die in diesem Reaktionsmedium erzielbaren hohen Ausbeuten in allen Stufen sowie die problemlos möglichen Phasentrennungen und die dadurch gegebene Möglichkeit, ohne Isolierung der Zwischenprodukte und ohne Lösungsmittelwechsel arbeiten zu können. Eine solche Arbeitsweise ermöglicht die Herstellung der Dioxazinverbindungen in einer technisch einfachen und wirtschaftlich vorteilhaften Weise.
o-Dichlorbenzol und andere chlorierte aromatische Lösungsmittel haben jedoch den Nachteil, daß die Produktionsabwässer AOX enthalten und daß bei den hohen Reaktionstemperaturen giftige und umweltschädliche Stoffe in Spuren entstehen und sich im Produktionskreislauf in dem regenerierten Lösungsmittel anreichern und auch im Produkt nachgewiesen werden können.
Mit zunehmender Berücksichtigung der Umweltbelange entstand deshalb ein Bedarf nach einem Herstellverfahren, das über alle Synthesestufen vorteilhaft in der Verfahrensdurchführung ist, d.h. keine Zwischenisolierungen und keine Lösungsmittelwechsel notwendig sind, aber im Gegensatz zu o-Dichlorbenzol ökologisch unbedenklich ist.

Die bisher vorgeschlagenen halogenfreien Reaktionsmedien haben Nachteile, die ihrer Verwendung in einem großtechnischen Verfahren zur Herstellung von Dioxazinverbindungen entgegenstehen.
Polare aprotische Lösungsmittel wie Chinolin und Dimethylformamid (JP-A 56-135 556) sind im Gegensatz zu o-Dichlorbenzol wasserlöslich und nicht für alle Reaktionsstufen der Synthese als Reaktionsmedium geeignet. Auch kann die Synthese in diesen Lösungsmitteln nicht ohne Zwischenisolierungen erfolgen. Bei der Verwendung dieser Lösungsmittel ist es erforderlich, vor dem Ringschluß das Kondensationsprodukt zu isolieren und das Reaktionsmedium zu wechseln, damit weitgehend wasserfrei gearbeitet werden kann und annehmbare Ausbeuten erzielt werden. Die Herstellung der Dioxazinverbindungen unter Verwendung dieser Lösungsmittel ist dadurch technisch aufwendig und unwirtschaftlich.

Monoalkyl-, Dialkyl-, Trialkylbenzole und Alkylnaphthaline (JP-A-7-331 097) und auch Alkane und Alkene (JP-A-7-331 098) haben ebenfalls Nachteile im Hinblick auf die technische und wirtschaftliche Herstellung der Dioxazinverbindungen. So müssen z.B. bei den Vorstufen wegen der schlechteren Löslichkeiten der Zwischenprodukte, der Nebenprodukte und des Chloranils in diesen Lösungsmitteln im Vergleich zu o-Dichlorbenzol wesentlich größere Reaktionsvolumina angewandt werden. Außerdem müssen die Phasen in größeren Behältern geschieden werden.

Daraus ergeben sich wesentlich schlechtere Raumausbeuten als bei einem Arbeiten in o-Dichlorbenzol. Darüberhinaus ergeben sich bei der Verwendung dieser Lösungsmittel Phasentrennprobleme, was zu langen Ansatzzeiten und somit zu schlechten Zeitausbeuten führt. Bei der Synthese ohne Zwischenisolierungen ist die schlechte Löslichkeit des überschüssigen Chloranils und der am Ende der Synthese angesammelten Nebenprodukte in diesen Lösungsmitteln ein weiterer schwerwiegender Nachteil. Die Dioxazinverbindungen müssen nach dem Abfiltrieren zusätzlich mit einem besser lösenden organischen Lösungsmittel gewaschen werden, um die Nebenprodukte und die nicht umgesetzten Edukte, die die anwendungstechnischen Eigenschaften verschlechtern, aus dem Produkt vollständig herauszulösen. Die Produktkristalle sind in der Regel wesentlich kleiner und schlechter ausgebildet als bei der Verwendung von o-Dichlorbenzol als Reaktionsmedium, weshalb sich bei dem notwendigen Waschen mit dem zusätzlichen polaren Lösungsmittel die Filtrierform drastisch verschlechtern kann. Das Arbeiten mit einem zweiten organischen Lösungsmittel erfordert außerdem einen zusätzlichen technischen Aufwand. Die in diesen halogenfreien Lösungsmitteln erzielbaren Ringschlußausbeuten sind nur mäßig. Aufgrund dieser Nachteile konnten auch diese vorgeschlagenen halogenfreien Lösungsmittel das für die technische Herstellung der Dioxazinverbindungen bisher bevorzugte o-Dichlorbenzol nicht ersetzten.

Die US 5,185,435 beschreibt eine Ammonolyse eines Säurechlorids mit einem aromatischen Amin in Anisol anstelle von o-Dichlorbenzol.

Es wurde nun überraschend gefunden, daß bestimmte, über 160°C siedende Arylalkylether als Reaktionsmedium für die gesamte Synthese der Dioxazinverbindungen besonders gut geeignet sind.

Die vorliegende Erfindung betrifft somit ein Verfahren zur Herstellung von Dioxazinen durch Ringschluß einer Verbindung der allgemeinen Formel (II) 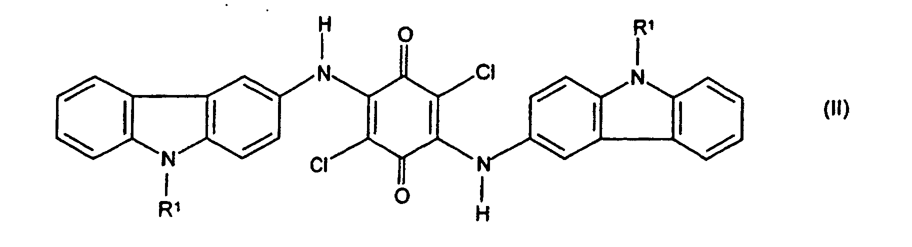 worin R¹ Wasserstoff oder (C₁-C₈)-Alkyl bedeutet, in Gegenwart eines Ringschlußmittels, dadurch gekennzeichnet, daß als Reaktionsmedium ein Arylalkylether der Formel (III) oder ein Gemisch aus Arylalkylethern der Formel (III) verwendet wird 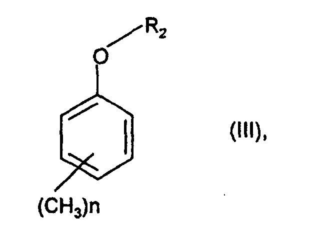 worin n eine ganze Zahl von 0 bis 2 bedeutet und R₂ Ethyl bedeutet, wenn n = 0 ist, oder R₂ Methyl bedeutet, wenn n = 1 oder 2 ist.

Bevorzugt ist n = 0 und R₂ = Ethyl (Phenetol) sowie n = 1 und R₂ = Methyl. Die Methylgruppe steht bevorzugt in m- oder p-Stellung zu der Etherfunktion. Besonders bevorzugt ist p-Kresolmethylether (4-Methylanisol).

Für R¹ stehendes (C₁-C₈)-Alkyl kann geradkettig oder verzweigt sein und beispielsweise für Methyl, Ethyl, n-Propyl, i-Propyl, n-Butyl, sek.Butyl, i-Butyl, tert.Butyl, Pentyl, Hexyl, Heptyl oder Octyl stehen. Bevorzugt ist Methyl, Ethyl oder Propyl, insbesondere Ethyl.

Die bei dem erfindungsgemäßen Verfahren entstehenden Dioxazin-Verbindungen können prinzipiell eine Struktur der Formeln (a), (b) oder (c) besitzen oder eine Mischung dieser Verbindungen sein:

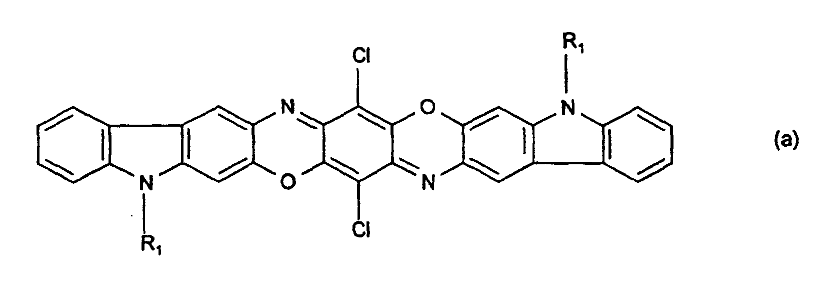

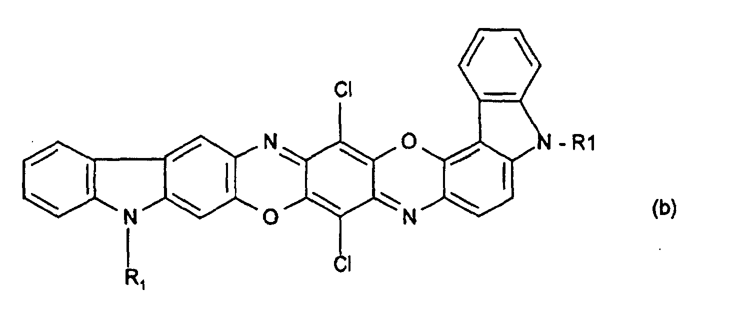

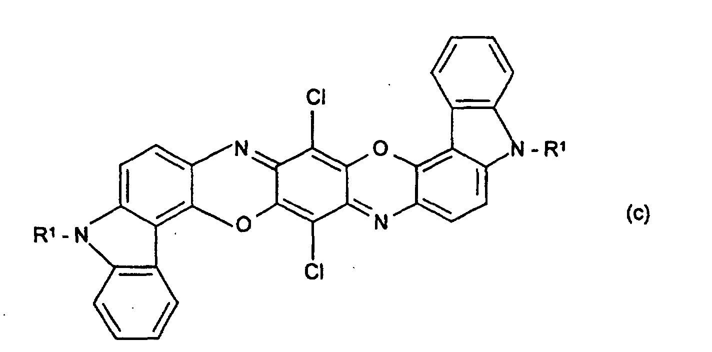

Der Arylalkylether wird zweckmäßigerweise in der 3- bis 20-fachen Gewichtsmenge, bevorzugt in der 5- bis 15-fachen Gewichtsmenge der Verbindung der Formel (II) eingesetzt.

Beispiele für Ringschlußmittel, die in dem erfindungsgemäßen Verfahren verwendet werden können, sind Benzolsulfonsäurechlorid, 4-Toluolsuhonsäurechlorid, nitro- oder chlorsubstituiertes Benzolsulfonsäurechlorid, Chloranil und Pyridin-N-oxid. Bevorzugtes Ringschlußmittel ist Benzolsulfonsäurechlorid oder 4-Toluolsulfonsäurechlorid.
Benzolsulfonsäurechlorid bzw. substituiertes Benzolsulfonsäurechlorid wird bevorzugt in Mengen von 0,5 bis 2,0 Mol, besonders bevorzugt 1,0 bis 1,5 Mol, pro Mol Verbindung der allgemeinen Formel (II), eingesetzt. Pyridin-N-oxid wird bevorzugt in einer Menge von 1 bis 3 Mol, insbesondere 1,2 bis 2,5 Mol, bezogen auf 1 Mol Verbindung der Formel (II), zugegeben. Chloranil wird bevorzugt in einer Menge von 5 bis 50 Mol-%, insbesondere 10 bis 40 Mol-%, bezogen auf die Verbindung der Formel (II), zugegeben.

Die Reaktionstemperatur liegt zweckmäßigerweise im Bereich von 140 bis 200°C, vorzugsweise 160 bis 190°C. Die Reaktion kann unter Normaldruck, unter Überdruck oder unter reduziertem Druck erfolgen. Bevorzugt ist die Reaktion unter Normaldruck. Die Reaktion benötigt in der Regel 1 bis 10 Stunden. Bevorzugt ist eine Reaktionszeit von 3 bis 7 Stunden.
Um die Dioxazinverbindungen in sehr reiner Form herzustellen, genügt es, nach dem Ringschluß das heiße Reaktionsgemisch, das einen erfindungsgemäßen Arylalkylether enthält, zu filtrieren und den Filterkuchen mit vorzugsweise heißem Arylalkylether zu waschen. Man erhält das reine Produkt in einer gut filtrierbaren Form mit hoher Ausbeute.

Beispielsweise kann am Ende der Reaktion das Reaktionsgemisch mit Arylalkylether verdünnt und heiß filtriert werden, vorzugsweise bei 100 bis 150°C. Die erhaltene Rohproduktpaste wird mit Arylalkylether bevorzugt heiß gewaschen und anschließend z.B. durch Blasen mit einem Inertgas von der Hauptmenge des anhaftenden Arylalkylethers befreit. Restmengen des Arylalkylethers können durch Wasserdampfdestillation oder Trocknung vollständig entfernt werden. Um die wasserlöslichen Nebenprodukte zu entfernen, wird das Produkt in der Regel noch mit Wasser angeschlämmt, gewaschen und getrocknet.

Die Verbindungen der allgemeinen Formel (II) können auch durch Kondensation von 3-Amino-N-alkyl-carbazolen der allgemeinen Formel (IV), 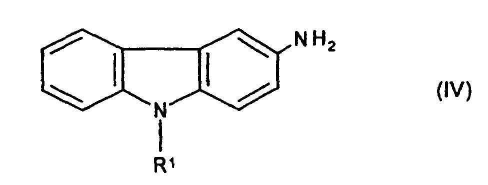 worin R¹ wie vorstehend definiert ist, mit Chloranil in einem oder mehreren der erfindungsgemäßen Arylalkylether und vorzugsweise in Gegenwart eines säurebindenden Mittels, wie z.B. Natriumacetat, Natriumhydrogencarbonat oder Natriumcarbonat, hergestellt werden. Dabei kann auch eine Lösung des 3-Amino-N-alkyl-carbazols der allgemeinen Formel (IV) in dem Arylalkylether, wie sie nach einer Reduktion des 3-Nitro-N-alkyl-carbazols in dem Arylalkylether anfällt, für die Kondensation mit Chloranil eingesetzt werden.

Die nach diesem Verfahren erhaltenen Verbindungen der allgemeinen Formel (II) können in Form der erhaltenen Suspension in dem Arylalkylether ohne Zwischenisolierung weiter zu den Dioxazinen umgesetzt werden.

Die vorliegende Erfindung betrifft daher auch ein Verfahren zur Herstellung von Dioxazinen durch Kondensation eines 3-Amino-N-alkyl-carbazols der Formel (IV), 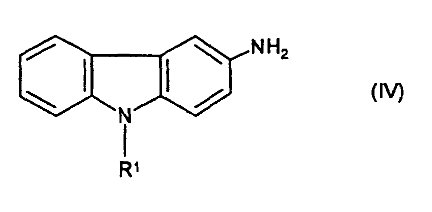 worin R¹ Wasserstoff oder (C₁-C₈)-Alkyl bedeutet, in einem Arylalkylether der Formel (III), mit Chloranil zur Verbindung der Formel (II) und anschließendem Ringschluß zu einem Dioxazin in Gegenwart eines Ringschlußmittels. Als Ringschlußmittel können die vorstehend genannten eingesetzt werden. Der Kondensationsschritt kann bei Temperaturen von 20 bis 90°C durchgeführt werden. Die Menge an Chloranil beträgt vorzugsweise 0,6 bis 0,9 Mol, bezogen auf 1 Mol eingesetztes 3-Amino-N-alkylcarbazol. Besonders vorteilhaft ist, daß das Kondensationsprodukt der allgemeinen Formel (II) nicht zwischenisoliert werden braucht und alle Reaktionsschritte in dem erfindungsgemäßen Arylalkylether erfolgen.

Die hervorragende Eignung der Arylalkylether für die Synthese der Dioxazinverbindungen war nicht vorhersehbar, da die Arylalkylether reaktionsfähige Verbindungen sind, die z.B. in den freien o- oder p-Stellungen leicht nitriert werden bzw. in Gegenwart von Säuren der Etherspaltung unterliegen (s. z.B. Beilstein, Band 6, 3. Ergänzungswerk, S. 1385). Es wurde gefunden, daß unter den Nitrierbedingungen, die eine hohe Ausbeute an 3-Nitro-N-alkylcarbazol ergeben, überraschenderweise weder eine signifikante Nitrierung des Arylalkylethers noch eine signifikante Spaltung der Etherbindung erfolgt. Ebenso erfolgte bei der mit säurebildenden Ringschlußmitteln bewirkten Ringschlußreaktion keine signifikante Etherspaltung der erfindungsgemäßen Arylalkylether. Im Vergleich mit Alkylbenzolen, Alkylnaphthalinen, Alkanen und Alkenen haben die erfindungsgemäßen Arylalkylether ein gutes Lösevermögen für die Edukte, Zwischenprodukte und Nebenprodukte der Synthese, so daß sehr gute Raum/ZeitAusbeuten möglich sind.

Die nachfolgenden Beispiele dienen der Erläuterung des erfindungsgemäßen Verfahrens.

### Beispiel 1:

In einem 0,5 Liter - Vierhalskolben mit KPG-Glasflügelrührer, Tropftrichter, Thermometer, Destillierbrücke und Ölbad werden 41,6 g 2,5-Dichlor-3,6-bis(9-ethyl-3-carbazolylamino)-1,4-benzochinon in 300 g 4-Methylanisol angeschlämmt. Man erwärmt die Anschlämmung auf 165°C und tropft bei 165°C in 15 Minuten 18,3 g Benzolsulfonsäurechlorid zu. Anschließend erhitzt man das Gemisch in 1 Stunde auf Siedetemperatur. Unter Abdestillieren wird 5 Stunden nachgerührt. Es destillieren etwa 100 ml ab. Das Reaktionsgemisch wird auf 150°C abgekühlt und filtriert. Der Filterkuchen wird mit 250 g 150°C heißem 4-Methylanisol gewaschen und bei 100 °C im Vakuum getrocknet. Man erhält 37,8 g der Dioxazin-Verbindung (91,6 % der Theorie). Die hohe Reinheit des Produktes zeigt sich in der Elementaranalyse, im IR-Absorptionsspektrum, dem Massenspektrum, dem Röntgendiffraktogramm und in den anwendungstechnischen Eigenschaften.

### Beispiele 2 und 3:

Es wird wie in Beispiel 1 gearbeitet mit dem einzigen Unterschied, daß anstelle von 4-Methylanisol 3-Methylanisol bzw. Phenetol verwendet wird. Man erhält die Dioxazin-Verbindung mit folgenden Ausbeuten:

| Beispiel | Reaktionsmedium | Ausbeute (% der Theorie) |
|---|---|---|
| 2 | 3-Methylanisol | 91,3 |
| 3 | Phenetol | 92,2 |

### Vergleichsbeispiel 1:

Es wird wie in Beispiel 1 gearbeitet mit dem einzigen Unterschied, daß anstelle von 4-Methylanisol o-Dichlorbenzol verwendet wird. Man erhält 34,8 g der Dioxazin-Verbindung (84,3 % der Theorie).

### Beispiel 4:

In einen 1 Liter-Vierhalskolben mit Rührer, Tropftrichter, Thermometer, Destillierbrücke und Ölbad gibt man 429,3 g einer 14,7 %-igen Lösung von 3-Amino-9-ethylcarbazol in 4-Methylanisol, die durch Reduktion der entsprechenden Nitroverbindung in 4-Methylanisol mit anschließendem Ausrühren, Waschen und Abtrennen der organischen Phase erhalten wurde. Man gibt 50 g 4-Methylanisol zu. Nach Zudosieren von 40 g Chloranil und 16 g Soda wird bei 30 bis 65°C gerührt, bis im DC kein 3-Amino-9-ethylcarbazol mehr zu sehen ist. Man gibt 200 g 4-Methylanisol zu, destilliert das Reaktionswasser unter Erhitzen auf 165°C ab, tropft in 15 Minuten bei 165°C 37 g Benzolsulfonsäurechlorid zu und heizt das Reaktionsgemisch in 1 Stunde auf Siedetemperatur. Anschließend wird 4 Stunden unter Abdestillieren nachgerührt. Es destillieren etwa 200 ml ab. Danach wird der Kolbeninhalt auf 150°C gekühlt und filtriert. Die Filtrationsgeschwindigkeit ist sehr hoch. Der Filterkuchen wird mit 500 g 150°C heißem 4-Methylanisol gewaschen, trocken geblasen und in Wasser ausgerührt. Nach dem Abdestillieren des 4-Methylanisol mit Wasserdampf wird filtriert, mit Wasser gewaschen und getrocknet. Man erhält 79,7 g der Dioxazin-Verbindung (90,1 % der Theorie). Die hohe Reinheit des Produktes zeigt sich in der Elementaranalyse, im IR-Absorptionsspektrum, dem Massenspektrum, dem Röntgendiffraktogramm und in den anwendungstechnischen Eigenschaften.

### Vergleichsbeispiel 2:

Es wird wie in Beispiel 4 gearbeitet mit dem einzigen Unterschied, daß anstelle von 4-Methylanisol o-Dichlorbenzol verwendet wird. Man erhält 74,0 g der Dioxazinverbindung (83,7 % der Theorie).

### Anwendungsbeispiel 1:

In einen Kunststoffbehälter, der mit 1400 Teilen zylindrischer Mahlkörper (Cylpebs®, Durchmesser 12 mm, Hersteller: Groh GmbH, Hof) zu 90 Vol-% gefüllt ist, werden nacheinander 22,5 Teile der Dioxazin-Verbindung, hergestellt gemäß einem der vorstehenden Beispiele, und 7,5 Teile wasserfreies Natriumsulfat eingebracht. Die Mischung wird 4 Stunden lang unter Schütteln auf einer Schwingmühle (Typ Vibratom; Hersteller: Siebtechnik Mühlheim) fein vermahlen. Danach wird das Mahlgut von den Mahlkörpern abgesiebt. Man erhält 25,9 Teile Mahlgut. In einem Rührgefäß werden 37,5 Teile Isobutanol (85 %ig) vorgelegt und nacheinander 2,5 Teile Ameisensäure 98 %ig und 0,38 Teile des Natriumsalzes eines Alkylphenolpolyglykolethersulfats zugegeben. Danach werden 25 Teile des obigen Mahlguts eingetragen und 20 Stunden bei 20-25°C gerührt. Während dieser Zeit werden noch 50 Teile Isobutanol 85 %ig zugesetzt. Anschließend werden 150 Teile Wasser zugegeben, 5 Stunden zum Sieden erhitzt und das Isobutanol durch Erhitzen auf 100 °C am Übergang azeotrop abdestilliert. Nach dem Abkühlen auf 60°C wird das Pigment abgesaugt, mit Wasser neutral und salzfrei gewaschen und bei 80°C getrocknet.
Man erhält 18,6 Teile Pigment, das im Alkydmelaminharz-Lack transparente und farbstarke violette Lackierungen mit rotem Farbton und hoher Reinheit liefert. Die Überlackierechtheit ist einwandfrei. Im Nitrocellulosetiefdruck werden transparente und farbstarke Drucke mit hoher Reinheit erhalten.

### Anwendungsbeispiel 2:

In einen Kunststoffbehälter, der mit 1400 Teilen zylindrischer Mahlkörper (Cylpebs®, Durchmesser 12 mm, Hersteller: Groh GmbH, Hof) zu 90 Vol-% gefüllt ist, werden 30 Teile der Dioxazin-Verbindung, hergestellt gemäß einem der vorstehenden Beispiele, eingebracht. Das Rohpigment wird 4 Stunden lang unter Schütteln auf einer Schwingmühle (Typ Vibratom; Hersteller: Siebtechnik Mühlheim) fein vermahlen. Danach wird das Mahlgut von den Mahlkörpern abgesiebt. Man erhält 25,9 Teile Mahlgut.
In einem Rührgefäß werden 37,5 Teile Isobutanol (85 %ig) vorgelegt und 1,25 Teile Ameisensäure 98 %ig zugegeben. Danach werden 25 Teile des obigen Mahlguts eingetragen und 20 Stunden bei 20-25°C gerührt. Während dieser Zeit werden noch 50 Teile Isobutanol 85 %ig zugesetzt. Anschließend werden 150 Teile Wasser zugegeben und das Isobutanol durch Erhitzen auf 100°C am Übergang azeotrop abdestilliert. Nach dem Abkühlen auf 60°C wird das Pigment abgesaugt, mit Wasser neutral gewaschen und bei 80°C getrocknet.
Man erhält 24,2 Teile Pigment, das in PVC transparente und farbstarke violette Ausfärbungen liefert. Die Ausblutechtheit ist sehr gut.

## Patentansprüche

1. Verfahren zur Herstellung von Dioxazinen durch Ringschluß einer Verbindung der allgemeinen Formel (II) 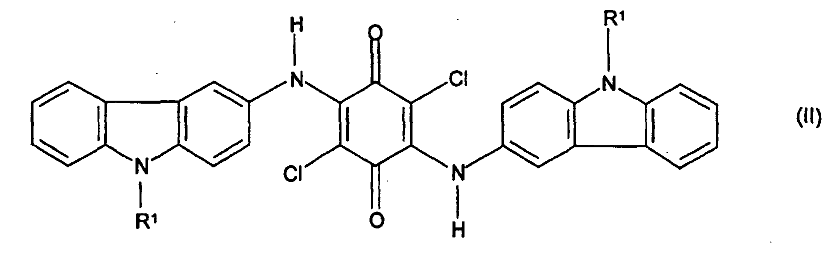 worin R¹ Wasserstoff oder (C₁-C₈)-Alkyl bedeutet, in Gegenwart eines Ringschlußmittels, **dadurch gekennzeichnet, daß** als Reaktionsmedium ein Arylalkylether der Formel (III) oder ein Gemisch aus Arylalkylethern der Formel (III) verwendet wird 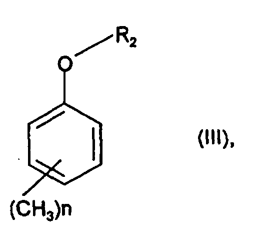 worin n eine ganze Zahl von 0 bis 2 bedeutet und R₂ Ethyl bedeutet, wenn n = 0 ist, oder R₂ Methyl bedeutet, wenn n = 1 oder 2 ist.

2. Verfahren zur Herstellung von Dioxazinen durch Kondensation eines 3-Amino-N-alkyl-carbazols der Formel (IV) 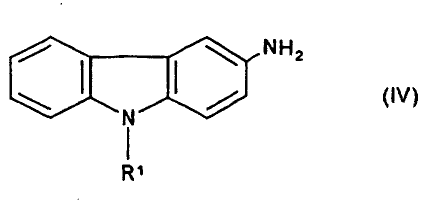 worin R¹ Wasserstoff oder (C₁-C₈)-Alkyl bedeutet, mit Chloranil zu einem Kondensationsprodukt der Formel (II) 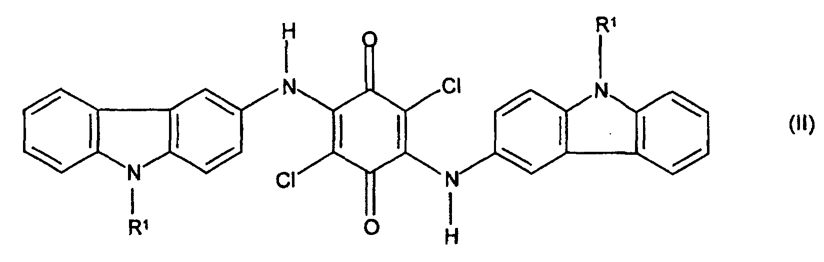 und anschließendem Ringschluß zum Dioxazin, **dadurch gekennzeichnet, daß** die Kondensation und der Ringschluß in einem Arylalkylether der Formel (III), oder in einem Gemisch aus Arylalkylethern der Formel (III), 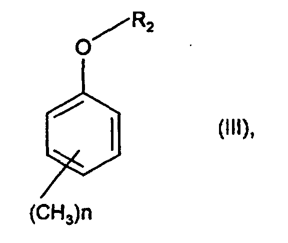 worin n eine ganze Zahl von 0 bis 2 bedeutet, und R₂ Ethyl bedeutet, wenn n = 0 ist, oder R₂ Methyl bedeutet, wenn n = 1 oder 2 ist, erfolgen.

3. Verfahren gemäß Anspruch 1 oder 2, **dadurch gekennzeichnet, daß** R¹ für Methyl, Ethyl oder Propyl steht.

4. Verfahren gemäß Anspruch 2, **dadurch gekennzeichnet, daß** die Verbindung der Formel (II) nicht zwischenisoliert wird.

5. Verfahren gemäß einem oder mehreren der Ansprüche 1 bis 4, **dadurch gekennzeichnet, daß** der Arylalkylether der Formel (III) Phenetol, 3-Methylanisol oder 4-Methylanisol ist.

6. Verfahren gemäß einem oder mehreren der Ansprüche 1 bis 5, **dadurch gekennzeichnet, daß** der oder die Arylalkylether der Formel (III) in der 3- bis 20-fachen, vorzugsweise 5- bis 15-fachen, Gewichtsmenge der Verbindung der Formel (II) eingesetzt werden.

7. Verfahren gemäß einem oder mehreren der Ansprüche 1 bis 6, **dadurch gekennzeichnet, daß** der Ringschluß bei einer Temperatur von 140 bis 200°C, vorzugsweise 160 bis 190°C, durchgeführt wird.

8. Verfahren gemäß einem oder mehreren der Ansprüche 1 bis 7, **dadurch gekennzeichnet, daß** das Ringschlußmittel Benzolsulfonsäurechlorid, 4-Toluolsulfonsäurechlorid, nitro- oder chlorsubstituiertes Benzolsulfonsäurechlorid, Chloranil oder Pyridin-N-oxid ist.

9. Verfahren gemäß einem oder mehreren der Ansprüche 1 bis 8, **dadurch gekennzeichnet, daß** das Reaktionsgemisch nach Beendigung des Ringschlusses bei einer Temperatur von 100 bis 150°C filtriert wird.

10. Verfahren gemäß Anspruch 9, **dadurch gekennzeichnet, daß** der erhaltene Filterkuchen mit einem Arylalkylether der Formel (III) gewaschen wird.

## Claims

1. A process for preparing dioxazines by ring closure of a compound of the formula (II) 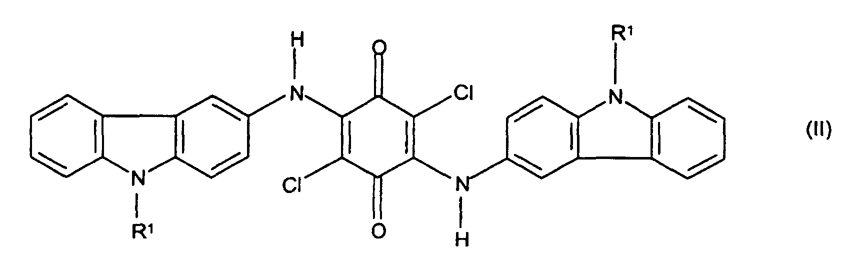 in which R¹ is hydrogen or C₁-C₈ alkyl in the presence of a ring closure agent, which comprises using as reaction medium an aryl alkyl ether of the formula (III) or a mixture of aryl alkyl ethers of the formula (III) 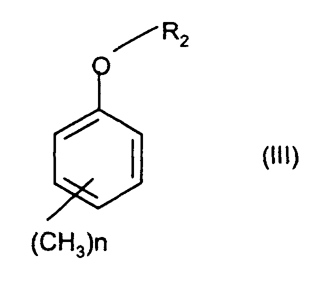 in which n is an integer from 0 to 2 and R₂ is ethyl if n is 0 or is methyl if n is 1 or 2.

2. A process for preparing dioxazines by condensing a 3-amino-N-alkylcarbazole of the formula (IV) 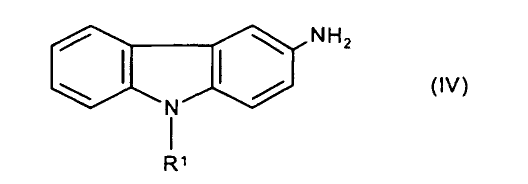 in which R¹ is hydrogen or C₁-C₈ alkyl with chloranil to give a condensation product of the formula (II) 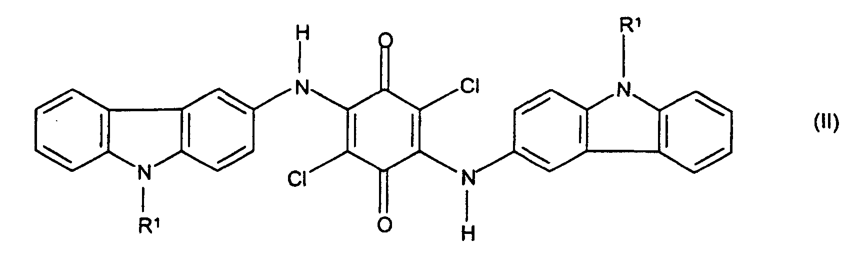 followed by ring closure to dioxazine, which comprises conducting the condensation and the ring closure in an aryl alkyl ether of the formula (III) or in a mixture of aryl alkyl ethers of the formula (III) 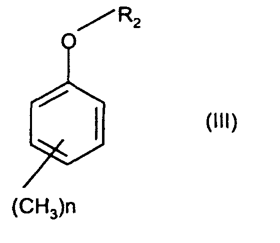 in which n is an integer from 0 to 2 and R₂ is ethyl if n is 0 or is methyl if n is 1 or 2.

3. The process as claimed in claim 1 or 2, wherein R¹ is methyl, ethyl or propyl.

4. The process as claimed in claim 2, wherein the compound of the formula (II) is not isolated.

5. The process as claimed in one or more of claims 1 to 4, wherein the aryl alkyl ether of the formula (III) is phenetole, 3-methylanisole or 4-methylanisole.

6. The process as claimed in one or more of claims 1 to 5, wherein the aryl alkyl ether(s) of the formula (III) is or are used in from 3 to 20 times, preferably from 5 to 15 times, the amount by weight of the compound of the formula (II).

7. The process as claimed in one or more of claims 1 to 6, wherein the ring closure is conducted at a temperature from 140 to 200°C, preferably from 160 to 190°C.

8. The process as claimed in one or more of claims 1 to 7, wherein the ring closure agent is benzenesulfonyl chloride, 4-toluenesulfonyl chloride, nitro- or chloro-substituted benzenesulfonyl chloride, chloranil or pyridine N-oxide.

9. The process as claimed in one or more of claims 1 to 8, wherein after the end of ring closure the reaction mixture is filtered at a temperature from 100 to 150°C.

10. The process as claimed in claim 9, wherein the filtercake obtained is washed with an aryl alkyl ether of the formula (III).

## Revendications

1. Procédé de préparation de dioxazines par cyclisation d'un composé de Formule générale (II) 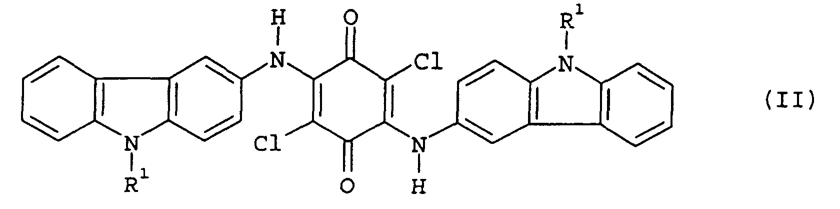 dans laquelle R¹ est un atome d'hydrogène ou un groupe alkyle en C₁ à C₈, en présence d'un agent de cyclisation, **caractérisé en ce qu'**on utilise en tant que milieu réactionnel un arylalkyléther de Formule (III) ou un mélange d'arylalkyléthers de Formule (III) 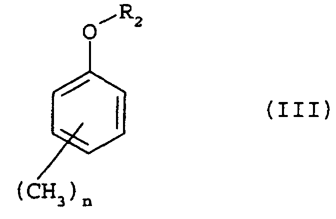 dans laquelle n est un nombre entier de 0 à 2 et R₂ est le groupe éthyle quand n vaut 0, ou R₂ est le groupe méthyle quand n vaut 1 ou 2.

2. Procédé de préparation de dioxazines par condensation d'un 3-amino-N-alkylcarbazole de Formule (IV) 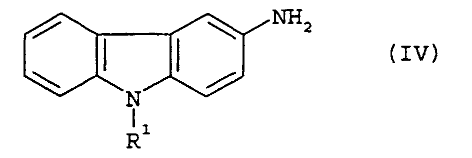 dans laquelle R¹ est un atome d'hydrogène ou un groupe alkyle en C₁ à C₈, avec du chloranile pour donner un produit de condensation de Formule (II) 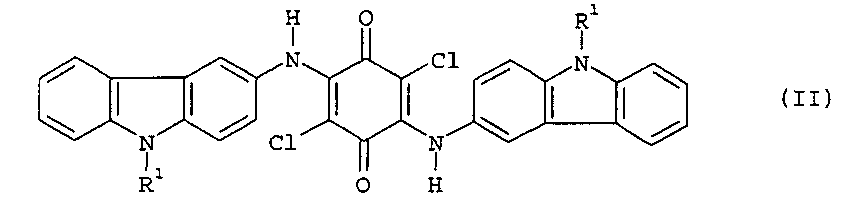 puis cyclisation pour donner la dioxazine, **caractérisé en ce que** la condensation et la cyclisation ont lieu dans un arylalkyléther de Formule (III), ou dans un mélange d'arylalkyléthers de Formule (III) 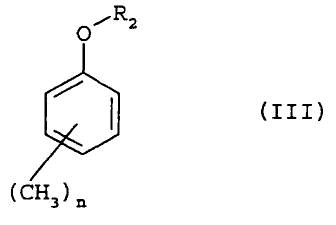 dans laquelle n est un nombre entier de 0 à 2, et R₂ est le groupe éthyle quand n vaut 0, ou R₂ est le groupe méthyle quand n vaut 1 ou 2.

3. Procédé selon la revendication 1 ou 2, **caractérisé en ce que** R¹ est le groupe méthyle, éthyle ou propyle.

4. Procédé selon la revendication 2, **caractérisé en ce que** le composé de Formule (II) n'est pas soumis à un isolement intermédiaire.

5. Procédé selon l'une ou plusieurs des revendications 1 à 4, **caractérisé en ce que** l'arylalkyléther de Formule (III) est le phénétole, le 3-méthylanisole ou le 4-méthylanisole.

6. Procédé selon l'une ou plusieurs des revendications 1 à 5, **caractérisé en ce que** le ou les arylalkyléthers de Formule (III) sont utilisés en une quantité pondérale 3 à 20 fois et de préférence 5 à 15 fois supérieure à celle du composé de Formule (II).

7. Procédé selon l'une ou plusieurs des revendications 1 à 6, **caractérisé en ce que** la cyclisation est mise en oeuvre à une température de 140 à 200°C et de préférence de 160 à 190°C.

8. Procédé selon l'une ou plusieurs des revendications 1 à 7, **caractérisé en ce que** l'agent de cyclisation est le chlorure de benzènesulfonyle, le chlorure de 4-toluènesulfonyle, le chlorure de benzènesulfonyle nitré ou chloré, le chloranile ou le N-oxyde de pyridine.

9. Procédé selon l'une ou plusieurs des revendications 1 à 8, **caractérisé en ce que** le mélange réactionnel, après la fin de la cyclisation, est filtré à une température de 100 à 150°C.

10. Procédé selon la revendication 9, **caractérisé en ce que** le gâteau de filtration obtenu est lavé avec un arylalkyléther de Formule (III).
